(19) Europäisches Patentamt
European Patent Office
Office européen des brevets

(11) Veröffentlichungsnummer: **0 300 285**
**A1**

(12) # EUROPÄISCHE PATENTANMELDUNG

(21) Anmeldenummer: 88110908.6

(51) Int. Cl.⁴: C07C 85/26 , C07C 87/62

(22) Anmeldetag: 08.07.88

(30) Priorität: 21.07.87 DE 3724018

(43) Veröffentlichungstag der Anmeldung:
25.01.89 Patentblatt 89/04

(84) Benannte Vertragsstaaten:
BE DE FR GB

(71) Anmelder: BAYER AG
Konzernverwaltung RP Patentabteilung
D-5090 Leverkusen 1 Bayerwerk(DE)

(72) Erfinder: Buysch, Hans-Josef, Dr.
Brandenburger-Strasse 28
D-4150 Krefeld(DE)
Erfinder: Hüllmann, Michael, Dr.
Siegfriedstrasse 41
D-6148 Heppenheim(DE)
Erfinder: Puppe, Lothar, Dr.
Am Weiher 10a
D-5093 Burscheid(DE)

(54) **Verfahren zur Trennung von Anilin-Derivaten.**

(57) Anilin-Derivate mit einer geringeren Zahl von am N-Atom gebundenen C-Atomen können von ihren Homologen mit größerer Zahl von am N-Atom gebundenen C-Atomen dadurch getrennt werden, daß man ein Gemisch der genannten Stoffe in flüssiger Phase mit Zeolithen vom Faujasit-Typ behandelt. In der flüssigen Phase befindet sich sodann ein Teil des Gemisches der genannten Stoffe in stark angereicherter Form. Bei der Regenerierung des Faujasits mit einer polaren Flüssigkeit nach der Behandlung des Gemisches und nach der Entfernung der flüssigen Phase, wird der andere Teil des Gemisches der Anilin-Derivate vom Faujasit gewonnen.

EP 0 300 285 A1

## Verfahren zur Trennung von Anilin-Derivaten

Die vorliegende Erfindung betrifft ein Verfahren zur Trennung von Anilin und Anilin-Derivaten voneinander, die am N-Atom eine verschiedene Zahl von gebundenen C-Atomen tragen.

Anilin und N-alkylierte Anilin-Derivate besitzen große Bedeutung in der Synthese wertvoller Zwischenprodukte. Beispielsweise ist N,N-Dimethylanilin ein wichtiges Ausgangsprodukt für die Darstellung von Farbstoffen der Triphenylmethan-Reihe (Michlers Keton, Methylviolett, Kristallviolett u.a.). Monomethylanilin wird außer zur Herstellung von Farbstoffen vornehmlich als Zwischenprodukt für pharmazeutische Präparate verwendet.

Bei der technischen Synthese N-alkylierter Anilin-Derivate aus Anilin und geeigneten Alkylierungsmitteln entstehen in der Regel Gemische verschieden hoch alkylierter Stoffe, die gegebenenfalls auch noch Ausgangsmaterial enthalten. Zur Trennung der entstandenen Stoffgemische sind aufwendige und teure Verfahren notwendig.

Beispielsweise entsteht bei der Methylierung von Anilin mit Methanol ein Gemisch von N-Monomethylanilin und N,N-Dimethylanilin und gegebenenfalls noch Anilin. Wegen der geringen Siedepunktdifferenz dieser Flüssigkeiten kann durch eine Vakuumdestillation nur bei sehr hohen Rücklaufverhältnissen eine befriedigende Trennung erzielt werden. Für höhere Reinheitsanforderungen an das N-Methylanilin muß zusätzlich noch mit Toluolsulfochlorid oder mit Phthalsäureanhydrid behandelt werden (Ullmanns Enzyklopädie der technischen Chemie, 3. Auflage, Band 3 (1953), S. 652).

Es bestand daher der Wunsch, solche aufwendigen und umständlichen Auftrennungen zu vereinfachen.

Es wurde nun überraschend gefunden, daß Anilin und Anilin-Derivate, die am N-Atom eine verschiedene Zahl von C-Atomen gebunden haben, mit Hilfe von Faujasit-Zeolithen sehr selektiv voneinander getrennt werden können.

Solche Trennungen an kristallinen Feststoffen sind in der Literatur kaum bekannt. Lediglich bei Toluidin-Isomeren ist in US 3.069.470 die Absorption an Zeolith CaX beschrieben, wobei eine gewisse para-Selektivität gefunden wurde. Auch US 4.480.129 beschreibt eine Methode zur Anreicherung von p-Toluidin mit Hilfe von mit Übergangsmetallen modifizierten X- und Y-Zeolithen, wobei allerdings komplizierte Lösungsmittelgemische mitverwendet werden müssen, die ihrerseits schwer abzutrennen sind. Trotz dieser aufwendigen Hilfsmaßnahmen wird der Trennfaktor kaum verbessert. Bei diesem Stand der Technik war nicht zu erwarten, daß die durchgeführten Trennungen die erfindungsgemäße außerordentlich hohe Selektivität aufweisen würden. Im Gegensatz zu den Toluidinen ist nämlich bei N-alkylierten Anilin-Derivaten um die (Alkyl)-C-N-Bindung eine freie Rotation und Änderung der Molekülgestalt möglich, während die einzelnen Toluidin-Isomeren unterschiedlich geformte starre Molekülgestalten besitzen, die auch eine unterschiedliche Anpassung an bestimmte Hohlräume in den Zeolithen und damit selektivere Trennungen erwarten lassen.

Es wurde nun ein Verfahren zur Trennung Anilin und Anilin-Derivaten voneinander, die am N-Atom eine verschiedene Zahl von gebundenen C-Atomen tragen, gefunden, das dadurch gekennzeichnet ist, daß man ein Gemisch der genannten Stoffe in flüssiger Phase mit Zeolithen vom Faujasit-Typ behandelt.

Im Regelfall betrifft die erfindungsgemäße Trennung ein Gemisch aus zwei Anilin-Derivaten. Auch in einem Gemisch von drei oder mehr Stoffen wird erfindungsgemäß jedoch eine so starke Trennwirkung erreicht, daß sie entweder die gestellten Anforderungen erfüllt oder zu einfacheren Stoffgemischen führt, die erfindungsgemäß oder herkömmlich weiter aufgetrennt werden können.

Im zu trennenden Gemisch aus Anilin und Anilin-Derivaten tragen die zu trennenden Stoffe eine verschiedene Zahl von am N-Atom gebundenen C-Atomen. Beispiele hierfür sind Stoffgemische, in denen verschiedene Alkyl-Substi tuenten am N-Atom vorliegen, wie ein Gemisch aus N-Methyl-anilin und N-Ethyl-anilin oder ein Gemisch aus N-Ethyl-anilin und N-Butyl-anilin. Weitere Beispiele sind Gemische von Stoffen mit einem verschieden hohen Alkylierungsgrad, beispielsweise ein Gemisch aus Anilin und N-Methyl-anilin oder ein Gemisch aus N-Ethyl-anilin und N,N-Diethyl-anilin. Ein typisches Dreiergemisch wäre beispielsweise ein Gemisch aus Anilin, N-Methyl-anilin und N,N-Dimethyl-anilin.

Die erfindungsgemäß zu trennenden Anilin-Derivate lassen sich durch die folgende Formel

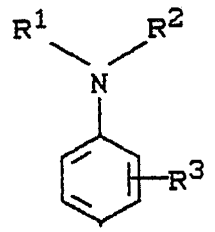

(I)

beschreiben, in der
$R^1$, $R^2$ und $R^3$ unabhängig voneinander für Wasserstoff oder $C_1$-$C_4$-Alkyl stehen
und worin weiterhin
$R^3$ auch noch Fluor, Chlor oder Brom oder $C_1$-$C_4$-Alkoxy bedeuten kann.

Im Sinne des erfindungsgemäßen Verfahrens sind jeweils mindestens zwei verschiedene unter die Formel (I) fallende Stoffe zur Trennung einzusetzen.

Unter die Formel (I) fallen beispielsweise folgende Verbindungen: Anilin, N-Methyl-anilin, N-Ethyl-anilin, N-Isopropyl-anilin, N-n-Propyl-anilin, N-n-Butyl-anilin, N-sec-Butyl-anilin, N-tert.-Butyl-anilin, N,N-Dimethyl-anilin, N,N-Diethyl-anilin, N,N-Di(n-propyl)-anilin, N,N-Di(isopropyl)-anilin, N,N-Di(n-butyl)-anilin, N,N-Di(sec-butyl)-anilin sowie die entsprechenden ortho-, meta- oder para-Toluidin-Derivate oder die entsprechenden Anilin-Derivate, die am aromatischen Kern noch eine ortho-, meta- oder para-ständige Ethyl-, Propyl- oder Butylgruppe tragen, die entsprechenden ortho-, meta-oder para-Chlor-, -Brom- oder -Fluor-anilin-Derivate oder o-, m-, p-Methoxy-, Ethoxy-anilinderivate.

In bevorzugter Form seien Anilin-Derivate der Formel

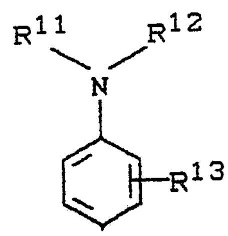

(II)

genannt, in der
$R^{11}$, $R^{12}$ und $R^{13}$ unabhängig voneinander für Wasserstoff oder $C_1$-$C_3$-Alkyl stehen.

In besonders bevorzugter Form seien Anilin-Derivate der Formel

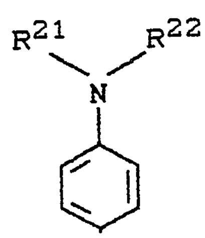

(III)

genannt, in der
$R^{21}$ und $R^{22}$ Wasserstoff, Methyl oder Ethyl bedeuten.

Die zu trennenden Anilin-Derivate liegen erfindungsgemäß in flüssiger Phase vor. Soweit ein zu trennendes Gemisch von solchen Anilin-Derivaten bei der gewählten Reaktionstemperatur eine Flüssigkeit darstellt, kann diese Flüssigkeit als solche erfindungsgemäß behandelt werden. Liegen die zu trennenden Anilin-Derivate als Feststoffe vor, können sie in einem geeigneten inerten Lösungsmittel gelöst werden. Auch flüssige Anilin-Derivat können mit solchen inerten Lösungsmitteln verdünnt werden.

Geeignete inerte Lösungsmittel sind aliphatische oder aromatische Kohlenwasserstoffe oder Halogenaromaten. In bevorzugter Weise werden inerte Lösungsmittel eingesetzt, die unter Normaldruck einen Siedepunkt unter 160° C haben. Beispiele für solche Lösungsmittel sind n-Pentant, n-Hexan, n-Heptan, iso-Octan, iso-Decan, iso-Dodecan, Cyclohexan, Methyl-cyclohexan, Benzol, Toluol, Xylol, Chlorbenzol oder

Brombenzol.

Das erfindungsgemäße Verfahren wird an Zeolithen vom Faujasit-Typ durchgeführt. Solche Faujasite kommen natürlich vor, können jedoch auch synthetisch hergestellt werden. In bevorzugter Weise werden synthetische Faujasite eingesetzt.

Faujasite für das erfindungsgemäße Verfahren können durch die Formel

$$(1.0\pm0,2)Me_{2/n}O \bullet Al_2O_3 \bullet (2\text{-}6)SiO_2 \bullet ZH_2O$$

beschrieben werden, in der
Me eines der weiter unten beschriebenen Kationen ist,
n die Ladung des Kations anzeigt und
Z eine Zahl von 1-10 ist.

Faujasite mit einem $SiO_2/Al_2O_3$-Verhältnis von 2 bis 3 werden allgemein als Zeolith X, solche mit einem $SiO_2$ $Al_2O_3$-Verhältnis von 3 bis 6 als Zeolith Y bezeichnet. Das Kation Me kann ausgetauscht werden. Faujasite und der Kationenaustausch sind bekannt und beispielsweise ausführlich in D. W. Breck, Zeolite Molecular Sieves, John Wiley & Sons, Inc., New York 1974, beschrieben worden.

Die Kationen Me können solche der Gruppen Ia, II, IIb, IVa, IVb, VIa, VIIa oder VIIIa sein, wenn man das Perio densystem in der Kurzperioden-Darstellungsweise zugrunde legt. Das Kation kann auch das Proton $H^+$ sein. In bevorzugter Weise enthält der Faujasit Kationen der Gruppen Ia, IIa, IVa oder IVb oder das Proton $H^+$. In besonders bevorzugter Weise enthält der Faujasit als Kationen die von Na, K, Cs, Ca, Ti, Sn oder das Proton $H^+$.

Der Faujasit wird in einer Menge von 10 - 500 Gew.-%, bevorzugt 100 - 350 Gew.-%, bezogen auf die Menge der zu trennenden Anilin-Derivate, eingesetzt.

Neben der überraschend hohen Trennleistung der Zeolithe vom Faujasit-Typ im Rahmen des erfindungsgemäßen Verfahrens muß die besonders gute Effektivität der Alkaliformen der Faujasite als völlig unerwartet bezeichnet werden, da Alkali-Zeolithe in der oben genannten US 4.480.129 als praktisch unbrauchbar eingestuft werden.

Das erfindungsgemäße Verfahren kann in einer üblichen und dem Fachmann bekannten Vorrichtung vorgenommen werden, beispielsweise in einem Rührgefäß für absatzweise Durchführung oder in einer Kolonnenapparatur für kontinuierliche Betriebsweise. Die Form und die Abmessungen dieser Reaktionsapparaturen können selbstverständlich optimiert werden.

Die erfindungsgemäße Behandlung der zu trennenden Gemische von Anilin-Derivaten wird bei einer Temperatur von 0°C bis 190°C, bevorzugt bei 10 bis 150°C, besonders bevorzugt bei 15 bis 120°C vorgenommen. Der Druck ist für das erfindungsgemäße Verfahren unkritisch; im allgemeinen wird bei Normaldruck gearbeitet.

Nach Beendigung des erfindungsgemäßen Verfahrens läßt man die flüssige Phase vom Faujasit ablaufen. Sie enthält einen Teil des Gemisches der zu trennenden Anilin-Derivate in stark angereicherter Form. Sofern ein inertes Lösungsmittel mitverwendet wurde, kann man dies destillativ abtrennen. Das inerte Lösungsmittel wird so ausgewählt, daß sein Siedepunkt von dem des angereicherten Gemischanteils genügend weit entfernt ist.

Auf dem Faujasit befindet sich der Rest des zu trennenden Ausgangs-Gemisches von Anilin und Anilin-Derivaten mit einer Anreicherung im umgekehrten Sinne, wie für den in der flüssigen Phase befindlichen Gemischanteil. Der Faujasit wird nach dem Ablassen der flüssigen Phase mit einer polaren Flüssigkeit in Kontakt gebracht, um ihn wieder zu regenerieren und erneut für das erfindungsgemäße Verfahren einzusetzen. Bei dieser Regenerierung wird der andere Teil des erfindungsgemäß getrennten Gemisches von Anilin-Derivaten gewonnen.

Polare Flüssigkeiten hierfür sind beispielsweise aliphatische Amine, Alkanole, Ether, Wasser oder deren Gemische. Dem Fachmann sind weitere grundsätzlich einsetzbare polare Flüssigkeiten bekannt; er wird jedoch relativ niedrig siedende polare Flüssigkeiten auswählen, die leicht von den vom Faujasit abzutrennenden Anilin und Anilin-Derivaten und vom Faujasit zu trennen sind. Beispiele solcher polarer Flüssigkeiten sind Butylamin, Diethylamin, Dipropylamin, Methanol, Ethanol, Diethylether, Isopropanol, n-Propanol, Wasser und ihre Gemische untereinander. Ein niedriger Siedepunkt solcher polarer Flüssigkeiten ermöglicht es auch, sie in der Dampfform mit dem Faujasit in Kontakt zu bringen. Der Faujasit wird daher nach dem Ablassen der flüssigen Phase mit einer solchen polaren Flüssigkeit oder ihrem Dampf bei einer Temperatur von 10 bis 200°C, bevorzugt 40 bis 150°C, besonders bevorzugt 60 bis 120°C, regeneriert. Will man die polare Flüssigkeit ohne wesentliche Verdampfung bei höheren Temperaturen innerhalb der genannten Bereiche anwenden, kann in einer dem Fachmann bekannten Weise auch unter Druck, beispielsweise unter dem Eigendruck des Systems, gearbeitet werden.

Als polare Flüssigkeit werden in bevorzugter Weise niedere Alkanole und/oder Wasser, in besonders bevorzugter Weise Methanol, Ethanol, Wasser oder ihre Gemische angewendet.

Die Vorteile des erfindungsgemäßen Verfahrens liegen in den außergewöhnlich hohen Selektivitäten, in der einfachen Verfahrensweise zur Trennung der genannten Anilin-Derivate und den niedrigen erforderlichen Verfahrenstemperaturen. Die beispielsweise in Säulenapparaturen kontinuierlich zu betreibende Behandlung der Gemische von Anilin-Derivaten und die ebenfalls kontinuierliche Regenerierung der Faujasite erbringt eine außerordentlich hohe Leistung pro Apparateeinheit.

Die folgenden Beispiele erläutern das erfindungsgemäße Verfahren näher, ohne es auf diese Beispiele einzuschränken.

Beispiel 1

Ein Gemisch aus 2,42 g N-Ethyl-anilin (44,8 Gew.-% des gesamten Gemisches) und 2,98 g N,N-Diethyl-anilin (55,2 Gew.-%) wurde in Cyclohexan gelöst, mit 16 g gepulvertem NaY-Zeolith in Berührung gebracht und 60 min bei 25 °C gerührt. Anschließend wurde die Lösung vom Zeolithen abfiltriert und gaschromatographisch untersucht. Nach Analyse enthielt das Filtrat einen Anteil von 4,2 % N-Ethyl-anilin und 95,8 % N,N-Diethyl-anilin. Gleiche Ergebnisse erzielt man beim Einsatz von Isododecan bzw. Benzol als Lösungsmittel.

Beispiel 2

Ein Gemisch aus 2,14 g N-Methyl-anilin (46,9 Gew.-% des gesamten Gemisches) und 2,42 g N,N-Dimethyl-anilin (53,1 Gew.-%) wurde in Cyclohexan gelöst, mit 16 g gepulvertem SnY-Zeolith in Berührung gebracht und 30 min bei 25 °C gerührt. Die gaschromatographische Analyse des Filtrats ergab einen Anteil von 15,9 Gew.-% N-Methyl-anilin und 84,1 Gew.-% N,N-Dimethyl-anilin.

Beispiel 3

Ein Gemisch aus 1,86 g Anilin (43,5 Gew.-% des gesamten Gemisches) und 2,42 g N-Ethyl-anilin (56,5 Gew.-%) wurde in Cyclohexan gelöst, mit 16 g gepulvertem NaX-Zeolith in Berührung gebracht und 12 h bei 25 °C gerührt. Die gaschromatographische Analyse des Filtrats ergab einen Anteil von 7,2 Gew.-% Anilin und 92,8 Gew.-% N-Ethyl-anilin.

Beispiel 4

Ein Gemisch aus 1,86 g Anilin (46,5 Gew.-% des gesamten Gemisches) und 2,14 g N-Methyl-anilin (53,5 Gew.-%) wurde in Cyclohexan gelöst, bei 25 °C mit 16 g gepulvertem NaX-Zeolith in Berührung gebracht und 30 min gerührt. Die gaschromatographische Analyse des Filtrats ergab einen Anteil von 15,4 Gew.-% Anilin und 84,6 Gew.-% N-Methyl-anilin.

Beispiel 5

Ein Gemisch aus 2,58 g N-Ethyl-anilin (47,8 Gew.-% des gesamten Gemisches) und 2,82 g N,N-Diethyl-anilin (52,2 Gew.-%) wurde in Cyclohexan gelöst, mit granuliertem 20 g NaY-Zeolith (Bindermaterial $SiO_2$) in Berührung gebracht und bei 25 °C 60 min unter Normaldruck gerührt. Nach gaschromatographischer Analyse des Filtrats ergab sich ein Anteil von 4,2 Gew.-% N-Ethylanilin und 95,8 Gew.-% N,N-Diethylanilin.

Anschließend wurde das im Zeolith eingeschlossene Gemisch der aromatischen Amine 2 h in siedendem Methanol desorbiert. Nach dem Abfiltrieren und Abdestillieren des Methanols wurde die zu erwartende Stoffmenge isoliert, die die Zusammensetzung 89 Gew.-% N-Ethyl-anilin und 11 Gew.-% N,N-Diethyl-anilin aufwies.

## Ansprüche

1. Verfahren zur Trennung von Anilin und Anilin-Derivaten voneinander, die eine verschiedene Zahl von gebundenen C-Atomen tragen, dadurch gekennzeichnet, daß man ein Gemisch der genannten Stoffe in flüssiger Phase mit Zeolithen vom Faujasit-Typ behandelt.

2. Verfahren nach Anspruch 1, dadurch gekennzeichnet, daß man synthetische Faujasite vom Type X oder Y einsetzt.

3. Verfahren nach Anspruch 1, dadurch gekennzeichnet, daß der Faujasit ein oder mehrere austauschbare Kationen der Gruppen Ia, IIa, IIb, IVa, IVb, VIa, VIIa oder VIIIa des Periodensystems in der Kurzperiodendarstellungsweise oder Protonen enthält.

4. Verfahren nach Anspruch 3, dadurch gekennzeichnet, daß der Faujasit Kationen der Gruppen Ia, IIa, IVa oder IVb oder Protonen enthält.

5. Verfahren nach Anspruch 4, dadurch gekennzeichnet, daß der Faujasit als Kationen die von Na, K, Cs, Ca, Ti, Sn oder $H^+$ enthält.

6. Verfahren nach Anspruch 1, dadurch gekennzeichnet, daß die Behandlung bei einer Temperatur von 0 °C bis 190 °C, bevorzugt 10 bis 150 °C, besonders bevorzugt 15 bis 120 °C, erfolgt.

7. Verfahren nach Anspruch 1, dadurch gekennzeichnet, daß der Faujasit nach der Behandlung des Gemisches der Anilin-Derivate und nach der Entfernung der flüssigen Phase mit einer polaren Flüssigkeit regeneriert wird.

8. Verfahren nach Anspruch 7, dadurch gekennzeichnet, daß als polare Flüssigkeit ein niederes Alkanol oder Wasser oder ein Gemisch von ihnen eingesetzt wird.

9. Verfahren nach Anspruch 7, dadurch gekennzeichnet, daß das Regenerieren bei einer Temperatur von 10 bis 200 °C, bevorzugt 40 bis 150 °C, besonders bevorzugt 60 bis 120 °C, durchgeführt wird.

Europäisches Patentamt

# EUROPÄISCHER RECHERCHENBERICHT

Nummer der Anmeldung: EP 88110908.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | US - A - 4 480 129 (PRIEGNITZ et al.)<br>* Spalte 8, Zeilen 16-21,50-57; Ansprüche *<br>-- | 1-3,6 | C 07 C 85/26<br>C 07 C 87/62 |
| A | US - A - 4 633 018 (ZINNEN)<br>* Ansprüche *<br>-- | 1-8 | |
| A | EP - A1 - 0 007 983 (HÜLS)<br>* Beispiel 7; Seite 3, Zeilen 26-35 *<br>-- | 1,3-8 | |
| A | GB - A - 128 372 (RINTOUL)<br>* Seite 4, Zeilen 38-43 *<br>---- | 1 | RECHERCHIERTE SACHGEBIETE (Int. Cl.4)<br>C 07 C 85/00<br>C 07 C 87/00 |

Der vorliegende Recherchenbericht wurde für alle Patentansprüche erstellt.

| Recherchenort | Abschlußdatum der Recherche | Prüfer |
|---|---|---|
| WIEN | 22-09-1988 | KÖRBER |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82